Europäisches Patentamt

European Patent Office

Office européen des brevets

(19)

(11) Publication number: **0 425 269 A2**

# EUROPEAN PATENT APPLICATION

(12)

(21) Application number: 90311654.9

(22) Date of filing: 24.10.90

(51) Int. Cl.⁵: **D01F 6/88**, //A61L15/00

(30) Priority: 25.10.89 US 427072

(43) Date of publication of application:
02.05.91 Bulletin 91/18

(84) Designated Contracting States:
AT DE ES FR GB IT NL

(71) Applicant: HOECHST CELANESE
CORPORATION
Route 202-206 North
Somerville, New Jersey(US)

(72) Inventor: KIM, Dai W.
44 Huron Drive
Chatham, New Jersey(US)
Inventor: Dibiase, Joseph J.
127 Idalroy Trail
Hopatcong, New Jersey(US)
Inventor: Pickton, Joseph
59 Woodbine Circle
New Providence, New Jersey(US)

(74) Representative: De Minvielle-Devaux, Ian
Benedict Peter et al
CARPMAELS & RANSFORD 43, Bloomsbury
Square
London WC1A 2RA(GB)

(54) **Superabsorbent polymer fibers and processes for their production.**

(57) This invention discloses the melt spun fiber blend of a conventional synthetic material and a superabsorbent polymer and a process for its production. In a preferred process the materials are comprised of blends selected from the group consisting of polyethylene, polypropylene, copolymers of a polyethylene, vinyl acetate, and cellulose acetates blended with a superabsorbent polymer. These fibers can be quite useful in wound dressings, athletic clothing, and other such uses where high water absorbance of a fiber is important.

EP 0 425 269 A2

## SUPERABSORBENT POLYMER FIBERS AND PROCESSES FOR THEIR PRODUCTION

(b) Cross Reference to Related Applications

NONE

(c) Background of Invention

1. Field of Invention

This invention relates to absorbent polymer articles and processes for their production. More particularly, the invention relates to synthetic fibers containing a superabsorbent polymer and processes for their production.

2. Prior Art

Recently there has been an interest in producing increasingly absorbent materials particularly for use in such products as sanitary napkins, diapers, disposable dust cloths, etc. The prior art materials used to form these products have been non-woven fabrics, papers, pulps, spongy urethane resins, natural sponges and the like. However, these materials exhibit relatively low absorbency, thus failing to satisfy the need for a low volume, high absorbent material.

There has also been interest in the production of woven materials with enhanced fluid absorbance. Conventional woven material, whether synthetic or natural, do not possess the high absorbency useful for applications such as surgical dressings, athletic wear, hygiene products, etc.

To solve some of these problems, new and improved highly absorbent products have been disclosed. For example, Japanese Ko-Kai No. 57-92,032 (1982) discloses a polyurethane foam containing a water absorbing resin. This highly water absorbent resin is particularly useful in the instant application. However, the use of the water absorbent resin in a fiber is not disclosed.

U. S. Patent Nos. 4,560,372 and 4,676,784 disclose disposable absorbent products in a layered structure comprised of an absorbent material mixed with a fibrous layer comprised of certain selected synthetic fibers such as polyester, polyethylene, polypropylene and the like. Although these patents disclose a mixture of synthetic fibers with fluid absorbent products, they do not disclose a process for the production of synthetic fibers which themselves contain a water absorbent material.

U. S. Patent No. 4,076,663 discloses a quite useful biodegradable, highly water absorbent polymer. However, once again the patent only discloses the mixing of this polymer in particulate or powder form in certain products such as sanitary napkins, diapers and other such products. See also U.S. Patent No. 3,669,103.

U. S. Patent Nos. 4,454,268, 4,337,181 and 4,133,784 disclose various types of films partially comprised of water absorbent polymers. However, these patents fail to disclose the combination of these water absorbent polymers with synthetic fibers.

Various types of products have been prepared wherein water absorbent materials are combined with conventional materials such as urethanes, amides, esters, wood fibers and the like. For example, U. S. Patent No. 4,767,825 discloses a process for the preparation of superabsorbent thermoplastic compositions and non-woven webs formed by melt blowing a superabsorbent thermoplastic material into a pulp fiber composition.

U. S. Patent Nos. 4,604,313 and 4,655,757 disclose processes for the preparation of layered materials containing superabsorbent products wherein the materials are prepared by a melt blown process on a continuously running belt. U. S. Patent No. 4,786,550 discloses a melt blown product containing therein absorbent materials such as cellulose fibers and superabsorbent materials for use as a seed bed. The process for the production of this product is somewhat similar to that disclosed in the '757 and '313 patents.

U. S. Patent No. 4,802,574 discloses an absorbent apparatus containing an absorbent strip sandwiched between layers of non-woven fabric to form a non-woven fabric combination. Contained within the absorbent material can be a superabsorbent product.

While many of these patents disclose products, including composite products, that contain fluid absorbent materials, none discloses a process for the production of synthetic materials containing superabsorbent products which are produced into fibers by a melt spinning process and, thereafter, woven into water absorbent products.

Accordingly, it is an object of this invention to prepare synthetic fibers containing a superabsorbent polymer.

It is the further object of this invention to prepare synthetic fibers containing a superabsorbent polymer by use of a melt spinning process.

It is a still further object of this invention to prepare fibers containing a superabsorbent polymer which are easy to produce and which retain many of the properties of the synthetic materials in addition to enhanced water absorbance.

These and other objects as well as the scope, nature and utilization of the invention will be apparent from the following detailed description.


(d) Summary of Invention


In accordance with the present invention there is provided a process for the production of a fiber containing a superabsorbent polymer comprising

(a) preparing a synthetic material mixture;

(b) blending with the synthetic material mixture superabsorbent polymer particulates; and

(c) melt spinning that blend to form a synthetic fiber containing a superabsorbent polymer.

Another embodiment of the instant invention is a superabsorbent synthetic fiber prepared by melt spinning a blend of a superabsorbent polymer particulate and a synthetic material.

The fibers produced by this process can be highly useful for the production of woven materials where high water absorbance is critical, such as in the production of medical dressings, clothing, athletic wear, food packaging, packaging material and other related uses.


(e) Detailed Description of Invention


The synthetic material that is combined with the superabsorbent polymer to form the superabsorbent synthetic fiber is selected from the group of materials which commonly use a melt spinning process as a means of forming fibers from said materials. Generally, these materials all are crystallizable and have a margin of not less than 30° C between their melting point and the temperature at which the molten polymer undergoes decomposition. In addition, certain polymers that are normally spun from solution because they decompose before melting sufficiently for melt extrusion can also be melt spun by this process if plasticized by incorporation of a high boiling solvent. For example, polyacrylonitrile containing 30% to 40% of dimethyl formamide can be melt spun at about 160° C and cellulose triacetate containing similar amounts of cyclohexanone is also melt spinnable. The plasticizer remains in the filament and may subsequently be removed by evaporation or washing.

The preferred materials chosen for the synthetic fiber of the instant invention include polyamides, such as nylon-6, nylon-11, nylon-6, 10 etc.; polyesters; polyolefins, such as polyethylene or polypropylene; polyvinylidene chloride copolymers, cellulose acetates and other synthetic polymers which are typically melt spun into fibers.

The synthetic materials chosen may also be a copolymer of the above mentioned materials such as polyethylene-vinyl acetate copolymers.

The more preferred materials chosen for the instant invention include polypropylene, polyethylene vinyl acetate copolymers and cellulose acetates.

The major limitation on the choice of the synthetic material is that its melt spinning temperature must be less than about 250° C since at that temperature the preferred superabsorbent polymer begins to decompose.

Once the appropriate synthetic material is chosen, it is prepared for the melt spinning process by conventional procedures. For example, in one preferred procedure, prior to melt spinning the synthetic material is ground into fine particles, i.e., less than about 50 microns. The grinding of these materials can be by any conventional grinding procedure commonly utilized in the industry for the specified synthetic material. It is important that these synthetic materials be finely ground so that the synthetic material and the superabsorbent polymer particulates can be well blended.

The superabsorbent particulates are next blended with the synthetic material. The superabsorbent polymers useful in this invention are water insoluble but water swellable polymers which are capable of absorbing many times their own weight of water or aqueous solutions. These superabsorbent polymers are polymers of water soluble acrylic or vinyl monomers which are slightly crosslinked with a polyfunctional reactant. Such polymers include polyvinylpyrrolidone, sulfonated polystyrene, polysulfoethyl acrylate, poly(2-hydroxyethyl-acrylate) polyacrylamide, polyacrylic acid, partial and complete alkali metal salts of polyacrylic acid, and the like. Also included are starch modified polyacrylic acids and hydrolyzed polyacrylonitrile and their alkali metal salts.

Useful superabsorbent polymers can be made by polymerizing acrylic acid and starch in an aqueous medium using a polyfunctional monomer, e.g., N, N-alkylene-bis-acrylamide, as the crosslinking agent. This process is described in U. S. Patent No. 4,076,663. Superabsorbent polymers can also be made as described in U. S. Patent No. 4,340,706 by the inverse polymerization of acrylic acid followed by crosslinking with a polyfunctional component, e.g., epichlorohydrin. Other superabsorbent polymers and processes for their manufacture are disclosed in U. S. Patent Nos. 4,654,039, 3,669,103 and 3,670,731. All of the aforesaid patents are hereby incorporated by reference.

The superabsorbent polymer particulates particularly useful in this invention are those described in U. S. Patent Nos. 4,076,663 and 4,340,706. These superabsorbent polymer particulates have a particle size of about 0.5 microns to about 450 microns and are capable of absorbing at least about 12 times their weight of aqueous fluid. In a preferred embodiment superior absorption capabilities exist where the size of the superabsorbent polymer particulates are less than about 5 microns, and most preferably less than about 1 micron. These superabsorbent polymer particulates swell when they absorb aqueous fluids. The particles maintain the approximate shapes and geometry they had before contact with the fluid but the dimensions thereof are greatly enlarged.

Once the synthetic material and the superabsorbent polymer particulates are prepared, they are mixed together in a conventional premelt spinning mixing container. The ratio of the synthetic material to the superabsorbent polymer particulate will depend upon the end result desired. For example, the greater the amount of the superabsorbent polymer particulate contained within the synthetic fiber, the greater the amount of fluid absorption of the fibers. However, when the percentage of the superabsorbent polymer within the resultant fiber becomes greater than about 30 percent, many of the desirable qualities of the synthetic fibers are degraded. Thus, in a preferred embodiment the percentage of the superabsorbent polymer in the blend of the superabsorbent polymer with the synthetic material should range from about 1% to about 30% and preferably from about 5% to about 20%.

Once the synthetic material and the superabsorbent polymer particulates are blended, they are melt spun by conventional melt spinning procedures. Preferably the blend is placed within a container and cold pressed under a pressure of at least about 5,000 lbs. psi to create a solid material. This solid material is then placed in the melt spinner and heated to the temperature normally associated with melt spinning of the synthetic material. As previously stated, it is critical that the temperature of this melt spinner be maintained at less than about 250° C or the preferred superabsorbent polymer will decompose. In a preferred embodiment the melt spinning temperature is less than about 200° C.

The material is spun by any conventional melt spinning procedure that is consistent with the melt spinning of the synthetic material. Depending on the particular synthetic material chosen, the fibers are spun into an inert environment such as nitrogen. After spinning, the fibers are cooled by air cooling and taken up on a conventional take-up device under pressure to draw the fibers. The amount of take-up drawing will depend on the synthetic material chosen and the preferred end use for the fibers. This drawing will encourage the correct orientation of the fibers and increase their strength. The melt flow rate of the synthetic fibers containing the superabsorbent polymer will also depend on the choice of the synthetic material.

The fibers prepared by this melt spinning process may be used in woven materials for such applications as wound dressings, athletic equipment or clothing and other uses consistent with the type of synthetic material chosen.

The following examples are given as specific illustrations of the invention. All parts and percentages are by weight unless otherwise stated. It is understood, however, that the invention is not limited to specific details set forth in these examples.

## Example 1

Eighteen grams of a low density, polypropylene polymer were mixed with two grams of a superabsorbent polymer. The superabsorbent polymer used was a graft copolymer of about 91% acrylic acid and 9% oxidized starch crosslinked with 0.1% N, N'-methylene-bis-acrylamide made by the process described in U. S. Patent No. 4,076,663. The polypropylene material was finely ground until the size of the particles were less than about 50 microns. The super absorbent polymer particulates chosen were less than 5 microns in particle size and preferably on average about 1 micron. The material was introduced into a 1 inch sq. cylinder and cold pressed at a pressure of 5,000 pounds per square inch. The cylinder was placed in a James Ram Spinning unit and heated to a temperature of about 225° C. This material was then taken up at a speed of 40 meters per minute.

The fibers produced by this process showed a denier of 3.70 dl/g, a tenacity of 0.6 grams/denier, an initial modulus of 18.0 grams and an elongation breakage percentage of 231%. The water absorbance percentage of this material was 128% of the weight of the fibrous material produced and 49% of the weight of the fibrous material in a 1% saline solution.

## Example 2

Similar fibers to those produced in Example 1 were prepared except the synthetic material chosen was a copolymer of polyethylene and an ethylene vinyl acetate. The overall copolymer/superabsorbent polymer composition comprised 20 grams with the superabsorbent polymer comprising 10% of the overall solid material.

Similar procedures were run to those shown in Example 1.

The fiber produced by this process showed a denier of 3.3 dl/g, a tenacity of 1.7 gm/denier, an initial modulus of 8.5 grams and an elongation at breaking percentage of 331%.

The water absorbance percentage by weight of this fiber was 180% of the weight of the fibrous material and 62% of the weight of the fibrous material in a 1% saline solution.

As is apparent from these examples, superabsorbent polymers can be combined with conventional synthetic materials to produce melt spun fibers which show great utility in areas where fluid absorbance is important such as for wound dressings, athletic clothing and for many other similar products.

## Claims

1. A process for the production of fibers containing a superabsorbent polymer comprising:
   (a) forming a blend of a synthetic material and superabsorbent polymer particulates; and
   (b) melt spinning that blend to form the synthetic fibers containing a superabsorbent polymer.

2. The process of Claim 1 wherein the melting point of the synthetic material is less than 250° C.

3. The process of Claim 1 or 2 wherein the synthetic material is selected from polyamides, polyesters, polyolefins, polyvinylidene chloride copolymers, and cellulose acetates.

4. The process of Claim 3 wherein the synthetic material is a polyethylene or a polypropylene.

5. The process of any of Claims 1-3 wherein the synthetic material is a combination of two or more synthetic polymers.

6. The process of Claim 5 wherein the synthetic material is a polypropylene, ethylene vinyl acetate copolymer.

7. The process of any of Claims 1-6 wherein the concentration of the superabsorbent polymer in the blend is from 1% to 30% by weight.

8. The process of Claim 7 wherein the concentration of the superabsorbent polymer in the blend is from 5% to 20% by weight.

9. The process of any of Claims 1-8 wherein the size of the superabsorbent polymer particulates is less than 5 microns.

10. The process of any of Claims 1-9 wherein the melt spinning is conducted at a temperature up to 250° C.

11. The process of any of Claims 1-10 such that the fibers absorb water in an amount at least 15 percent of the weight of the superabsorbent polymer contained within the fibers.

12. A process for the production of fibers containing a superabsorbent polymer comprising:
   (a) preparing ground synthetic material particulates wherein the particulates are less than 50 microns and wherein the synthetic material is selected from polyamides, polyesters, polyolefins, polyvinylidene chloride copolymers, and cellulose acetates;
   (b) blending with that synthetic material superabsorbent polymer particulates; and
   (c) melt spinning that blend at a temperature less than 250° C to form synthetic fibers containing a superabsorbent polymer.

13. A process for the production of fibers containing a superabsorbent polymer comprising:
   (a) preparing ground polyethylene particulates wherein the particulate size is less than 50 microns;
   (b) blending with those ground polyethylene particulates superabsorbent polymer particulates comprising graft copolymers of acrylic acid and oxidized starch; and
   (c) melt spinning that blend at a temperature less than 250° C to form polyethylene fibers containing a superabsorbent polymer.